# FASCICULE DE BREVET EUROPEEN

(11) **EP 1 509 177 B1**
(45) Date de publication et mention de la délivrance du brevet: **10.08.2011**
(21) Numéro de dépôt: 03756027.3
(22) Date de dépôt: 30.05.2003
(51) Int. Cl.: A61F 5/00

(54) **ANNEAU GASTRIQUE**
MAGENRING
GASTRIC RING

(30) Priorité: 31.05.2002 FR 0206705
(43) Date de publication de la demande: 02.03.2005
(73) Titulaire: Textile Hi Tec, 81270 Labastide Rouairoux (FR)
(72) Inventeur: BOUSTANI, Roland, F-45000 Orléans (FR); HOUARD, William, F-81270 Labastide Rouairoux (FR); BERRET, Philippe, 34580 St. Paul et Valmalle (FR)
(74) Mandataire: Hennion, Jean-Claude
(86) Numéro de dépôt international: PCT/FR2003/001631
(87) Numéro de publication internationale: WO 2003/101352

(56) Documents cités:
- EP-A- 0 611 561
- EP-A- 1 463 468
- EP-A- 1 463 469
- FR-A- 2 778 847
- FR-A- 2 799 118
- US-A- 3 368 344
- US-A- 4 955 913
- US-A- 5 152 770

## Description

La présente invention concerne un anneau gastrique, dénommé également anneau de gastroplastie ou bande gastrique ou organe de constriction gastrique.

La pose d'un anneau gastrique fait partie des techniques développées pour lutter contre l'obésité massive dont est victime un nombre croissant d'individus dans les pays développés.

Comparativement à d'autres techniques, telles que la dérivation gastrojujénale (BPG ou By Pass Gastric) ou la dérivation bilio-pancréatique ou la gastroplastie verticale calibrée laparoscopique (VGB ou Vertical Banding Gastroplastie), la pose d'un anneau gastrique présente l'avantage de ne pas modifier l'anatomie du patient. Son principe est de réduire le diamètre de l'ouverture de l'estomac, créant une poche antérieure de faible volume, de sorte que le patient a une sensation de satiété après avoir ingéré une quantité minime d'aliments.

Depuis son apparition en 1986, plusieurs modèles d'anneaux gastriques ont été proposés, implantables par chirurgie laparoscopique. Tous ont cependant une structure sensiblement similaire. Il s'agit d'une bande relativement étroite, d'une certaine longueur, dans un matériau suffisamment souple pour pouvoir être replié en boucle, ladite bande étant pourvue de moyens de fermeture entre ses extrémités distale et proximale. De plus des moyens de réglage, actionnables de l'extérieur, permettent de régler le diamètre intérieur de l'anneau gastrique, une fois posé.

On connaît le document EP 1 463 469 relevant sous l'art 54(3) CBE, qui porte sur un anneau gastrique comprenant une bande apte à entourer une paroi de l'estomac. On connaît également le document US 4 955 913 qui divulgue un lien chirurgical pour la réalisation d'hystérectomie féline et canine. On connait aussi le document EP 1 463 468 (relevant sous l'art 54 (3) CBE), qui porte sur un anneau gastrique comprenant un element longiforme deformable en boucle compose des maillons.

Dans l'anneau connu sous la dénomination Lap Band® ou LAGB®, le caractère réglable est obtenu grâce à une section intérieure gonflable de l'anneau, ladite section étant celle qui est destinée à être en contact avec l'estomac. Un tube de liaison relie ladite section gonflable à une pièce dite d'injection. Lors de la pose de l'anneau, le tube de liaison et la pièce d'injection sont implantés à demeure dans le corps du patient, la pièce d'injection étant facilement accessible. Lors de la pose, le gonflage de l'anneau est minimum. Le réglage du diamètre se fait en injectant le fluide de gonflage dans la pièce d'injection.

L'anneau gastrique connu sous la dénomination SAGB® (Swedish Adjustable Gastric Band) diffère principalement du Lap Band® par le système de fermeture qui consiste dans un système de languette à cran de sûreté, l'anneau se refermant en tirant sur cette languette jusqu'à atteindre le cran de sûreté. L'anneau connu sous la dénomination Héliogast® se distingue des deux premiers également par son système de fermeture qui met en oeuvre un verrouillage hydraulique.

Malgré son efficacité reconnue , en terme de perte de poids, la technique de l'anneau gastrique n'est pas sans inconvénient, notamment du fait des complications qu'elle peut entraîner.

Certaines de ces complications sont liées à la présence de la pièce d'injection, qui peut présenter des défauts , et à la tubulure de liaison qui peut présenter des ruptures. Selon une étude récente, plus de 8% des complications observées sont dues à ces deux causes. Le changement de la pièce d'injection nécessite une réintervention sous anesthésie générale. Une rupture de la tubulure de liaison nécessite une réintervention par laparoscopie.

La pièce d'injection peut également être le siège de suppurations aiguës ou chroniques.

Enfin , sur le plan esthétique , l'implantation de la pièce d'injection laisse une cicatrice de 5 à 6 cm.

D'autres complications connues de la technique de l'anneau gastrique consistent dans un phénomène de migration de l'anneau dans la paroi gastro-gastrique et également dans un phénomène de glissement de la paroi de l'estomac à travers l'anneau. Même si certaines complications de ce type peuvent intervenir rapidement après l'implantation de l'anneau, la plupart apparaissent à moyen terme, après plusieurs années.

Le but que s'est fixé le demandeur est de proposer un anneau gastrique qui pallie tout ou partie des inconvénients précités.

Il s'agit d'un anneau gastrique qui comprend , de manière connue, un élément longiforme déformable en boucle entre une partie d'extrémité distale et une partie d'extrémité proximale et des moyens de fermeture aptes à replier sur lui-même l'élément longiforme et à fixer les parties d'extrémités distale et proximale, une fois que celles-ci ont été rapprochées lors du repliement de l'anneau.

De manière caractéristique , selon l'invention, au moins l'élément longiforme est dans un matériau résorbable.

Le caractère résorbable de l'élément longiforme , qui constitue le corps de l'anneau gastrique, permet de limiter dans le temps la présence dudit anneau et donc des complications qui sont liées à cette présence à moyen et long termes. De plus le caractère non-définitif de la pose de l'anneau , selon l'invention, aura un impact psychologique important sur le choix du patient. Après une période probatoire limitée dans le temps, il aura la possibilité d'arrêter l'expérience ou , s'il le souhaite , de procéder à une nouvelle implantation soit définitive soit de nouveau limitée dans le temps.

De préférence l'élément longiforme est dans un matériau lentement résorbable sur une période de l'ordre de ou inférieure à 2 ans, et encore plus préférentiellement comprise entre 16 et 24 mois.

D'une part cette période de temps correspond à celle pendant laquelle il y a perte de poids effective, précédant la stabilisation du poids.

D'autre part c'est après cette période que la présence de l'anneau gastrique entraîne le plus de risques en terme de migration , de perforation ou d'infection, en terme de dilatation progressive de l'oesophage et en terme de troubles du comportement. Ces risques sont donc supprimés du fait de ce choix du temps de résorption.

L'anneau est essentiellement composé de matériau(x) biodégradable(s) d'origine naturelle ou synthétique, tels que :
- polylactide, polyglycolide, poly ε-caprolactone
- polyhydroxybutyrate, polyhydroxyvalérate
- polycarbonates
- cellulose, polysaccharides, amidon...
   ...leurs homopolymères, leurs copolymères et leurs dérivés.

De préférence le matériau constitutif de l'élément longiforme est un poly-α-hydroxy acide, qui est une famille de polymères biorésorbables.

Avantageusement, parmi les poly-α-hydroxy acides connus, on met en oeuvre un polyacide lactique , plus particulièrement un poly (L-lactide-co-D,L-lactide). Au stade ultime de la dégradation, on obtient de l'acide lactique ou de l'acide glycolique, respectivement métabolite et pré-métabolite naturel du corps humain. Ces acides sont oxydés dans l'organisme en acide pyruvique qui est lui-même métabolisé en gaz carbonique et en eau via le cycle des acides tri-carboxyliques. L'élément longiforme de l'anneau gastrique, dans un tel matériau, est donc totalement résorbé dans le corps du patient. Cette résorption se fait à une vitesse variable en fonction de différents facteurs tels que la composition chimique et le taux de cristallinité, la masse moléculaire et le degré de polymérisation. Par exemple plus le polyacide lactique est cristallin plus sa résorption sera lente.

Selon un mode de réalisation de l'anneau gastrique de l'invention, l'élément longiforme est constitué d'un ensemble de maillons juxtaposés. Il est donc composé d'un maillon proximal, d'un maillon distal et de maillons intermédiaires.

Chaque maillon a par exemple une forme globalement polyédrique avec une face intérieure, destinée à être en contact avec l'estomac lors de la pose de l'anneau, et deux faces latérales internes dont au moins les portions situées à proximité de la face intérieure sont obliques et radialement convergentes en sorte que , lors de la fermeture de l'anneau, toutes lesdites portions radiales des faces latérales internes sont appliquées les unes contre les autres et les faces intérieures des maillons constituent une surface sensiblement continue de constriction.

Pour pouvoir être introduits par trocart, il est nécessaire que tous les maillons intermédiaires soient solidarisés les uns aux autres. De préférence , chaque maillon intermédiaire comporte , en saillie sur une première face latérale interne , un épaulement formant axe de pivotement entre ledit maillon et un premier maillon adjacent et , sur la seconde face latérale interne , un évidement formant logement pour l'épaulement d'un second maillon adjacent, l'épaulement et l'évidement étant dimensionnés pour être solidarisables par emboîtement.

Les moyens de fermeture doivent permettre le repliement sur lui-même en boucle de l'élément longiforme et la solidarisation des parties d'extrémités distale et proximale, notamment du maillon distal et du maillon proximal.

Dans un mode de réalisation, les moyens de fermeture comportent au moins un lien qui est solidaire d'au moins les parties d'extrémités proximale et distale.

Avantageusement le ou lesdits liens sont résorbables avec une période de résorption supérieure à la période de résorption de l'élément longiforme.

Selon cette disposition particulière, l'anneau gastrique garde son efficacité même lorsque l'élément longiforme et notamment les maillons ont perdu une partie importante de leur résistance mécanique.

Eventuellement le (ou lesdits) lien n'est pas résorbable, pour autant que sa présence dans le corps du patient après résorption totale de l'élément longiforme ne soit pas un inconvénient, ce qui est le cas lorsque le lien se retrouve à l'état libre et non serré autour de l'estomac ; c'est donc le cas lorsque le lien ne lie pas ensemble les parties d'extrémités distale et proximale.

Dans un mode préféré de réalisation, mettant en oeuvre des maillons juxtaposés pour former l'élément longiforme, chaque maillon comporte deux trous traversants, formant dans l'ensemble des maillons deux alignements de trous, pour un double passage d'un seul lien , depuis le maillon proximal jusqu'au maillon distal pour le premier alignement puis du maillon distal jusqu'au maillon proximal pour le second alignement. Il suffit, pour obtenir le repliement de l'élément longiforme , de se saisir des deux bouts libres du lien , d'exercer une traction sur ceux-ci en bloquant en position le maillon proximal pour rapprocher le maillon distal du maillon proximal, puis d'effectuer un noeud entre les deux bouts libres du lien pour maintenir l'élément longiforme dans sa configuration en boucle.

De préférence pour maintenir l'élément longiforme dans sa configuration repliée en boucle, les moyens de fermeture comportent des moyens de verrouillage formés , au moins en partie , dans les parties d'extrémités proximale et distale de l'élément longiforme, notamment les maillons proximal et distal.

Par exemple une partie d'extrémité comporte un élément mâle d'emboîtement tandis que l'autre partie d'extrémité comporte un élément femelle d'emboîtement. L'élément mâle peut être notamment un plot et l'élément femelle peut être notamment un évidement. Cet évidement, s'agissant de maillons , est par exemple creusé dans une face latérale externe du maillon proximal ou distal. Lors de la pose de l'anneau, le repliement des maillons est effectué sous la forme d'une spire avec le maillon distal venant au niveau et sur le côté du maillon proximal , il suffit alors à l'opérateur de rapprocher les deux dits maillons pour emboîter le plot de l'un dans l'évidement de l'autre et obtenir ainsi le verrouillage de l'anneau.

Dans le mode de réalisation où les moyens de fermeture comportent un lien , avantageusement la partie d'extrémité proximale, éventuellement le maillon proximal , comporte une tubulure de passage des deux bouts libres du lien ; cette tubulure est de préférence conformée pour sa fixation sur un ancillaire d'introduction de l'anneau. Elle peut notamment être taraudée pour sa fixation par vissage sur ledit ancillaire. Dans ce cas il est préférable également de prévoir un capuchon d'obturation de la tubulure, percé d'un trou traversant pour le passage des deux bouts libres du lien, ledit capuchon recouvrant la portion taraudée de la tubulure, après la pose de l'anneau et le retrait de l'ancillaire.

La présente invention sera mieux comprise à la lecture de la description qui va être faite d'un exemple de réalisation d'un anneau gastrique résorbable, formé par la juxtaposition de maillons en poly-acide lactique, munis d'un lien de fermeture non résorbable, illustré par le dessin annexé dans lequel :
La figure 1 est une vue schématique en perspective de l'anneau dans sa position repliée et verrouillée,
La figure 2 est une vue schématique en perspective d'un maillon intermédiaire,
La figure 3 est une vue schématique en perspective du maillon proximal,
La figure 4 est une vue schématique en perspective du maillon distal et

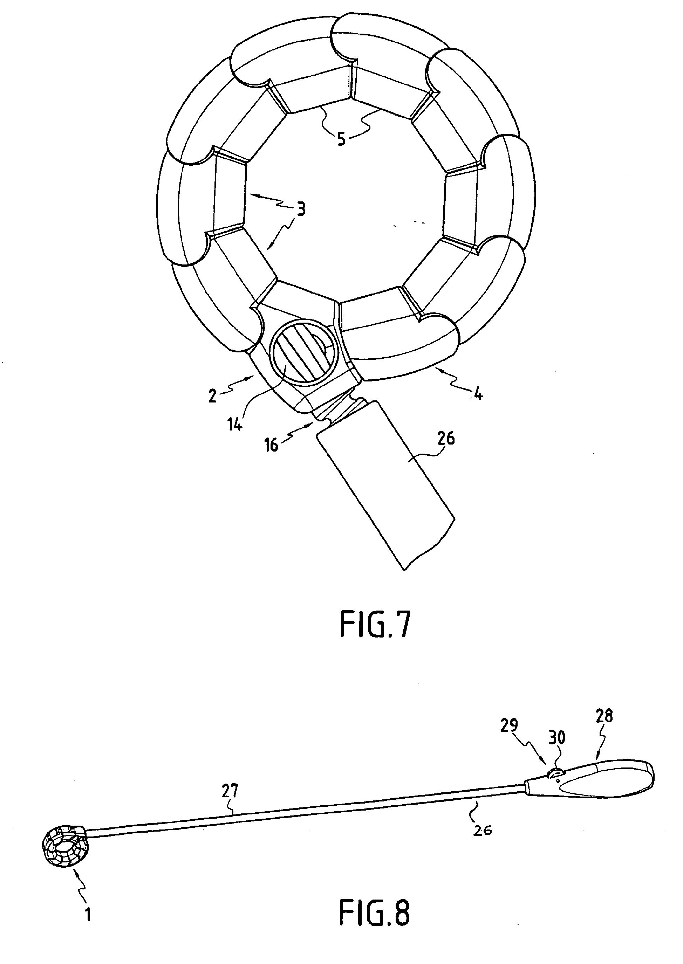

Les figures 5 , 6 et 7 illustrent les étapes de mise en place de l'anneau gastrique, avec une représentation partielle de l'ancillaire et
La figure 8 est une vue schématique en perspective de l'anneau gastrique à l'état replié porté par son ancillaire.

L'anneau gastrique de la présente invention se caractérise par son caractère temporaire , du fait de la biorésorbabilité de l'élément longiforme qui constitue le corps principal dudit anneau. Ainsi, contrairement aux anneaux gastriques connus , celui de la présente invention est destiné à n'assurer la constriction de l'estomac que pour une période prédéterminée , sans nécessiter de nouvelle intervention à l'issue de cette période. Le choix du matériau résorbable permet de régler la durée de cette période. Une période de 16 à 24 mois est en tout cas de l'ordre ou inférieure à 2 ans est considérée comme préférable. Ceci n'est toutefois pas exclusif et , en fonction des cas particuliers , il pourra être possible de proposer des anneaux gastriques pour une utilisation temporaire de durée plus courte ou plus longue.

Dans l'exemple de réalisation qui va être décrit ci-après , il s'agit d'un anneau gastrique non-réglable en dimension. Ceci n'est toutefois pas limitatif de la présente invention. Un anneau gastrique réglable , ayant une structure analogue à celle des anneaux connus rappelés ci-dessus, peut présenter une durée de fonctionnement prédéterminée , grâce à la biorésorbabilité de son élément longiforme, en choisissant pour la constitution de cet élément longiforme , un matériau qui présente le caractère biorésorbable souhaitable. Toutefois , dans ce cas , il subsiste les inconvénients liés à la présence des moyens particuliers permettant le réglage du diamètre de l'anneau , à moins de choisir également pour ces moyens de les réaliser dans un matériau également biorésorbable.

La résorption de l'anneau, alliée à son caractère éventuellement non réglable, se traduit également par une baisse des coûts, du fait de la réduction ou suppression de certains contrôles post-opératoires , et donc par une baisse des risques de complications liées à ces contrôles , qu'il s'agisse des radiographies de l'estomac et des ponctions répétées.

L'anneau gastrique 1 qui va être plus particulièrement décrit ci-après a son élément longiforme qui est constitué de maillons juxtaposés , chaque maillon étant une pièce monobloc formée dans un matériau rigide biorésorbable et également biocompatible.

Dans un mode précis de réalisation, le matériau biorésorbable choisi est un poly-α-hydroxy acide, plus précisément un polyacide lactique. Ce polymère perd progressivement ses propriétés mécaniques jusqu'à sa complète résorption. Il présente un squelette qui est sensible à l'hydrolyse. Sa dégradation se produit du fait de la conjonction de deux phénomènes , à savoir la pénétration de l'eau dans la matrice qui entraîne la formation de chaînes courtes de polymères par coupure de liaison ester et la migration ou la diffusion de ces chaînes courtes vers l'extérieur. S'agissant de pièce monobloc rigide telle que les maillons du présent anneau gastrique, le coefficient de diffusion de l'eau à travers la matrice polymère est supérieur au coefficient de migration des produits d'hydrolyse. De ce fait on observe une dégradation plus rapide au coeur du maillon qu'à sa périphérie, de sorte que l'anneau gastrique reste efficace pendant une période plus longue du fait que c'est sa périphérie interne qui détermine la constriction sur l'estomac.

Au stade ultime de la résorption du polyacide lactique , on obtient de l'acide lactique ou de l'acide glycolique qui sont respectivement métabolite et pré-métabolite naturels du corps humain. Ces acides lactique et glycolique sont oxydés dans l'organisme en acide pyruvique, lui-même métabolisé en gaz carbonique et en eau , selon le cycle des acides tri-carboxyliques.

Ainsi l'anneau gastrique de la présente invention est totalement résorbé dans le corps du patient , cette résorption se faisant à une vitesse variable en fonction des caractéristiques du matériau résorbable retenu.

S'agissant de poly-α-hydroxy-acide , les facteurs qui influent sur le temps de résorption sont notamment la composition chimique et le taux de critallinité , la masse moléculaire et le degré de polymérisation, la morphologie de la pièce et en particulier s'il s'agit d'une pièce rigide monobloc ou d'un film , le taux de monomère résiduel et la présence d'éventuelles autres impuretés , les dimensions de la pièce , le mode de stérilisation , le site d'implantation du polymère dans le corps... Parmi les facteurs les plus importants , il convient de noter le taux de cristallinité. Plus un poly-α-hydroxy-acide est cristallin et plus sa résorption sera lente.

S'agissant d'un polyacide lactique, il est possible d'influer sur la cristallinité en utilisant un copolymère à partir de monomère L-lactide et de monomère D,L-lactide. De préférence on met en oeuvre un copolymère avec un rapport molaire L-lactide/D,L-lactide qui est de l'ordre de 70/30 à 80/20. Un tel polyacide lactique perd ses propriétés mécaniques au bout de quelques mois mais n'est résorbé dans le corps qu'après une durée nettement plus longue, fonction notamment du site d'implantation, qui peut être de l'ordre de deux ans , ce qui est une durée adéquate comme cela a été expliqué ci-dessus.

L'anneau gastrique 1 est , dans l'exemple illustré , composé de dix maillons juxtaposés , à savoir un maillon proximal 2, huit maillons intermédiaires 3 et un maillon distal 4.

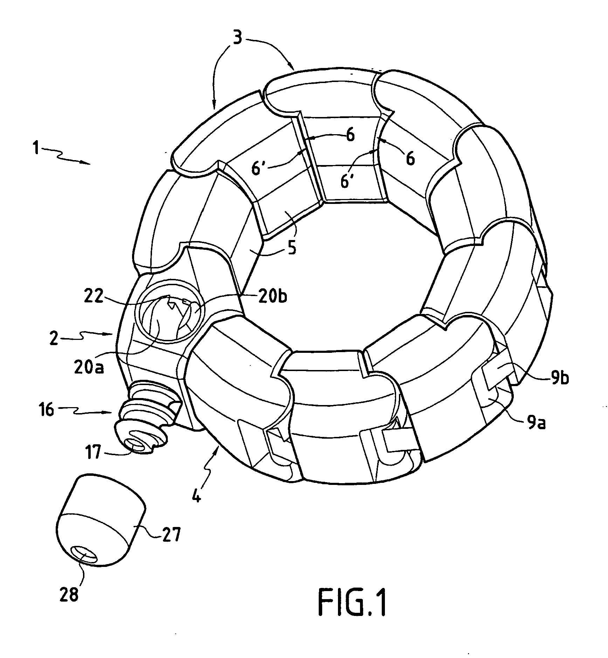

Sur la figure 1 , l'anneau gastrique 1 est dans sa configuration de constriction autour de l'estomac, l'ensemble des maillons juxtaposés ayant été replié en forme de boucle , les maillons proximal 2 et distal 4 ayant été verrouillés l'un par rapport à l'autre , ce repliement en forme de boucle et ce verrouillage étant obtenus grâce à des moyens de fermeture plus précisément décrits ci-après.

Il est à noter que dans l'exemple 1 illustré , la surface intérieure de l'anneau gastrique 1 qui est en contact avec l'estomac a une configuration polygonale , à dix faces , et non circulaire. Cette disposition n'est pas de nature à altérer l'efficacité de l'anneau en terme de constriction. Il aurait également été possible d'atténuer cette configuration polygonale en donnant aux faces intérieures 5 de chaque maillon 2,3,4 une certaine concavité.

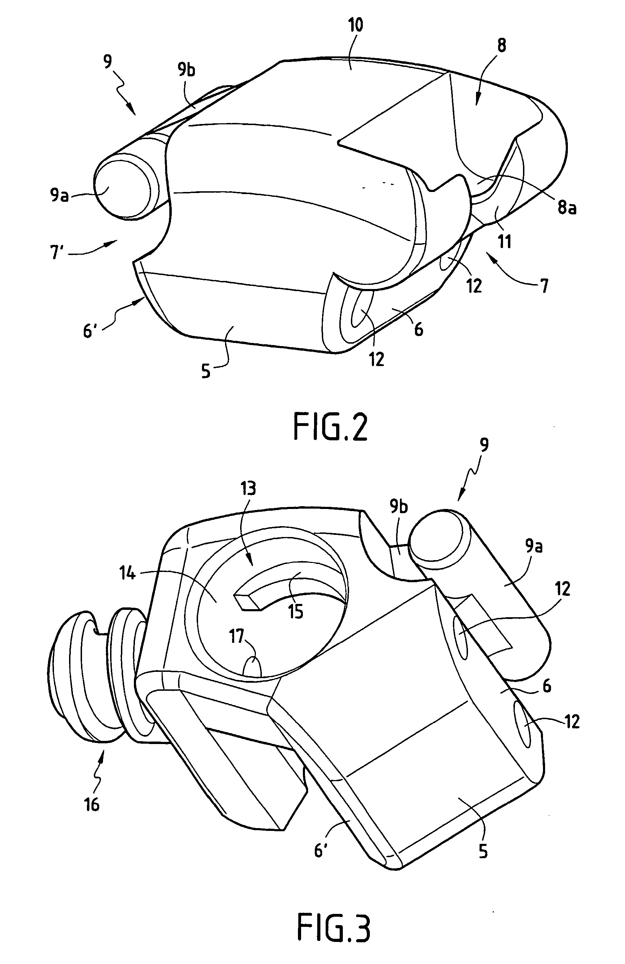

Sur la figure 2 est représenté un maillon intermédiaire 3, vu en perspective. Ce maillon intermédiaire 3 a une forme globalement polyédrique dont trois faces sont déterminantes , à savoir la face intérieure 5 et les deux faces latérales internes 7,7', plus précisément les portions 6, 6' de ces deux faces latérales internes 7,7' qui sont situées à proximité de la face intérieure 5. La face intérieure 5 , visible sur la figure 1, est destinée à être en contact avec l'estomac lors de la pose de l'anneau 1. Les portions 6, 6' des deux faces latérales internes 2,7' sont obliques et radialement convergentes en sorte que , lors de la fermeture de l'anneau 1, toutes lesdites portions radiales 6,6' , y compris celles des maillons proximal 2 et distal 4 , desdites faces latérales internes sont appliquées les unes contre les autres. Ainsi les faces intérieures 5 de tous les maillons constituent la surface, sensiblement continue, de constriction de l'estomac.

On comprend que pour un anneau gastrique 1 composé de dix maillons , tels que celui qui est donné en exemple, l'angle α formé entre les deux portions 6, 6' des deux faces latérales internes 7,7' doit être de 36°.

Etant donné que l'ensemble des maillons, constituant l'élément longiforme de l'anneau gastrique 1, doit pouvoir être introduit à l'état rectiligne à l'aide d'un trocart , il est nécessaire que chaque maillon soit solidaire l'un de l'autre. Pour ce faire, chaque maillon intermédiaire 3 comporte des éléments d'articulation et de pivotement , constitués par un évidement 8 et par un épaulement 9. L'évidement 8 est creusé dans la partie supérieure du maillon, à l'opposé de la portion 6 et débouche dans la face extérieure 10 et dans la face latérale interne 7. L'épaulement 9 est en saillie par rapport à l'autre face latérale interne 7' dans la zone supérieure du maillon , opposée à la portion 6'. La conformation de l'évidement 8 et de l'épaulement 9 est telle que l'épaulement 9 d'un maillon intermédiaire 3 donné est apte à pénétrer dans l'évidement 8 du maillon qui est immédiatement adjacent au maillon donné , tandis que l'évidement 8 du maillon donné est apte à recevoir l'épaulement 9 du maillon qui lui est immédiatement adjacent.

Dans l'exemple illustré à la figure 2 , l'épaulement 9 est constitué d'une partie cylindrique 9a , constituant l'axe de pivotement pour le repliement des différents maillons lors de la fermeture de l'anneau et d'une partie de raccordement 9b entre ladite partie cylindrique 9a et la face latérale interne 7' du maillon 3.

L'évidement 8 forme une cavité dont le fond 8a, semi-cylindrique, est destiné à accueillir, par emboîtement, la partie cylindrique 9a d'un épaulement 9. Cette cavité 8 est ouverte à la fois sur la face extérieure 10 du maillon 3, c'est-à-dire celle qui est à l'opposé de la face intérieure 5, et également sur la face latérale interne 7. Cette seconde ouverture 11 permet le passage de la portion 9b de raccordement de l'épaulement 9 du maillon adjacent lors de l'articulation des différents maillons entre-eux. Chaque maillon , notamment le maillon intermédiaire 3, comporte deux trous traversants 12, qui traversent le maillon de part en part entre les deux portions 6, 6' radiales des faces latérales internes 7, 7'.

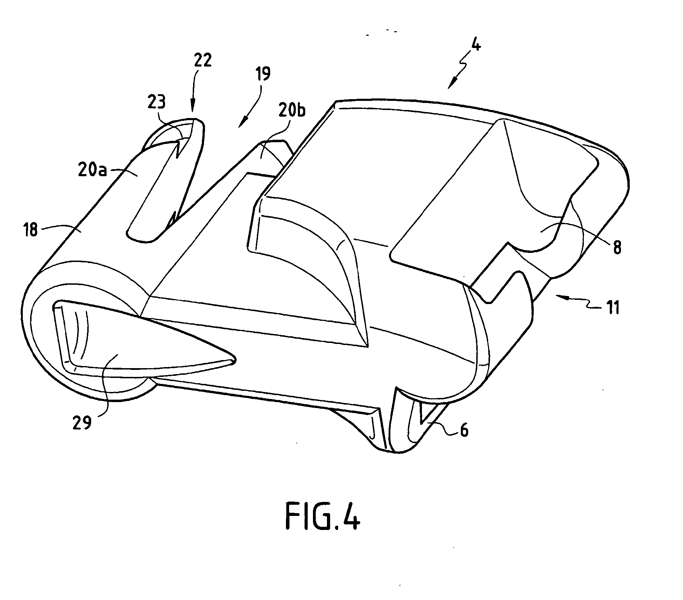

On a représenté aux figures 3 et 4 respectivement le maillon proximal 2 et le maillon distal 4. Sur ces figures , les mêmes références ont été utilisées pour les parties qui sont communes au maillon intermédiaire 3.

Le maillon proximal 2 comporte comme tous les maillons intermédiaires 3 une face inférieure 5 , deux trous traversants 12 et un épaulement 9 formé d'une partie cylindrique 9a et d'une partie de raccordement 9b. Par contre , il ne comporte pas d'évidement 8 , le verrouillage avec le maillon distal 4 ne se faisant pas par l'intermédiaire d'une articulation à pivotement mais par des moyens de verrouillage spécifiques , formés dans le maillon proximal 2 et le maillon distal 4 et aptes à coopérer l'un avec l'autre.

Le moyen de verrouillage du maillon proximal 2 consiste dans un élément femelle d'emboîtement 13, formé d'un évidement transversal 14, cylindrique , dont la paroi intérieure est munie d'une butée 15, partiellement annulaire , venant en saillie vers l'intérieur de l'évidement 14.

Le maillon proximal 2 comporte également une tubulure 16, percée par un trou traversant 17 et dont la surface périphérique extérieure est taraudée, pour permettre le vissage du maillon proximal 2 sur un ancillaire 26 lors de la pose de l'anneau gastrique 1.

Il est à noter que les deux trous traversants 12, contrairement au maillon intermédiaire 3 et également au maillon distal 4, ne débouchent pas sur la seconde face latérale interne 6' mais dans l'évidement transversal 14, en regard du trou traversant 17.

Le maillon distal 4 comporte , comme tous les maillons intermédiaires 3, un évidement 8 avec une ouverture 11, ainsi que deux trous traversants 12, non visibles sur la figure 4.

Les moyens de verrouillage du maillon distal 4 avec le maillon proximal 2 consistent en un plot transversal 18 de configuration globalement cylindrique , avec une encoche 19 délimitant ledit plot 18 en deux branches , une première branche d'extrémité 20a et une seconde branche 20b raccordée au reste du maillon distal 4 par une partie intermédiaire 21. Comme cela apparaît clairement à l'examen de la figure 4, l'encoche 19 n'étant que sur une partie de la largeur du maillon 4 , la branche d'extrémité 20a présente une certaine flexibilité par rapport à la seconde branche 20b. L'extrémité 22 des deux branches 20a, 20b est taillée en biseau pour permettre de faciliter la pénétration du plot 18 dans l'évidement 14 du maillon proximal 2. De plus la branche flexible 20a présente , vers ladite extrémité en biseau 22 une rainure partiellement annulaire 23 , destinée à recevoir la butée 15 du maillon proximal 2.

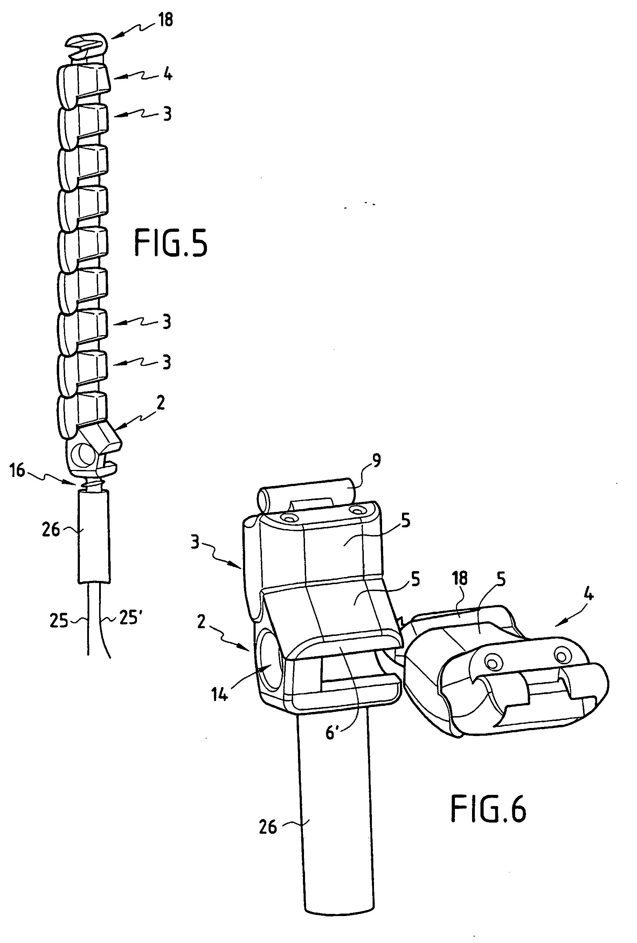

En plus des moyens de verrouillage qui viennent d'être décrits, les moyens de fermeture de l'anneau gastrique 1 comportent un moyen permettant le repliement, sous forme d'une boucle, de l'ensemble des maillons juxtaposés , de telle sorte que le maillon distal 4 puisse venir se placer à la hauteur et juste à côté du maillon proximal 2 en vue de leur verrouillage mutuel. Ce moyen de repliement consiste dans un lien qui passe dans les trous traversants 12 de tous les maillons et également dans le trou traversant 17 de la tubulure 16 du maillon proximal 2. Plus précisément lorsque les maillons sont alignés sous une forme rectiligne comme cela est illustré à la figure 5 , les trous traversants 12 constituent deux alignements parallèles , pour le lien 24. Ce lien 24 passe d'abord dans l'un des deux alignements , depuis le maillon proximal 2 jusqu'au maillon distal 4 puis dans le second alignement depuis le maillon distal jusqu'au maillon proximal 2. Les deux bouts libres 25, 25' dudit lien 24 sont passés ensemble dans le trou traversant 17 de la tubulure 16 et également dans l'ancillaire 26, qui est vissé sur la tubulure 16 et qui est destiné à permettre la préhension de l'anneau gastrique 1 dans sa configuration rectiligne de la figure 5. Bien sûr lors de l'introduction de l'anneau gastrique dans cette configuration, celui-ci est disposé à l'intérieur d'un trocart non représenté sur la figure 5.

Etant donné que le lien 24 est décalé par rapport à la portion cylindrique 9a des épaulements 9, faisant office d'axe de pivotement , la traction exercée par le praticien sur les deux bouts libres 25, 25' provoque un effet de traction sur le maillon distal 2 , ce qui amène celui-ci à pivoter autour de son axe de pivotement jusqu'à ce que sa face latérale interne 6 vienne en appui contre la face latérale interne 6' du maillon intermédiaire adjacent , lequel est amené alors à pivoter... et ainsi de suite jusqu'à repliement complet de l'ensemble des maillons sous forme d'une spire, non encore fermée.

Sur la figure 6 , on a représenté partiellement cette spire , avec le maillon distal 4 se trouvant au niveau et juste à côté du maillon proximal 2. Dans cette disposition, le plot 18 du maillon distal 4 se trouve en vis-à-vis de l'évidement transversal 14. Il suffit au praticien, avec un instrument adapté , de rapprocher l'un de l'autre ces deux maillons en sorte de faire pénétrer le plot 18 dans l'évidement 14. Il est aidé en cela par la forme en biseau 22 des deux branches 20a, 20b du plot 18. Lors de cette pénétration et grâce à la forme en biseau 22, la branche flexible 20a passe au-delà de la butée 15 jusqu'à ce que celle-ci pénètre dans la rainure 23. On a ainsi le verrouillage complet et irréversible des deux maillons proximal 2 et distal 4 et donc de l'anneau gastrique 1 autour de l'estomac. Le praticien peut alors retirer l'ancillaire 26 et confirmer la configuration refermée de l'anneau gastrique 1 en effectuant un noeud serré entre les deux bouts libres 25, 25' du lien 24 au niveau de la tubulure 16. Eventuellement il peut réaliser ce noeud après avoir recouvert la tubulure taraudé 16 à l'aide d'un capuchon d'obturation 27 , percé d'un trou 28 pour le passage des deux bouts libres 25, 25' du lien 24.

Pour faciliter l'opération de rapprochement des maillons proximal 2 et distal 4 , en vue de leur verrouillage , au cours de laquelle le praticien utilise un matériel du type pince, le maillon distal 4 comporte un rainurage latéral 29 sur la face du maillon distal 4 qui se trouve au niveau du plot 18, à l'opposé de l'encoche 19. En positionnant les extrémités des deux branches de la pince d'une part dans ce rainurage 29 du maillon distal 4 et d'autre part dans l'ouverture 14 du maillon proximal 2 , le praticien assure un positionnement précis , face à face des deux maillons distal 4 et proximal 2 , les deux branches 20a et 20b du plot 18 étant en regard de l'évidement 14. Il lui suffit alors de rapprocher les deux branches de la pince pour faire rentrer le plot 18 dans l'évidement 14 jusqu'au verrouillage comme indiqué ci-dessus.

La présente invention n'est pas limitée au mode précis de réalisation qui vient d'être décrit. Le nombre , la configuration et la dimension des maillons sont fonction du diamètre de constriction du l'estomac. Etant donné que dans la version décrite , ce diamètre n'est pas réglable , il sera proposé au praticien plusieurs modèles d'anneaux gastriques de manière à ce qu'il puisse sélectionner celui qui correspond le mieux au cas qu'il doit traiter.

Bien que cela n'a pas été explicitement dit dans la description ci-dessus, il est à noter que toute configuration d'anneau ne doit pas présenter de bord tranchant , ceci pour éviter toute lésion de la paroi gastrique. De plus la largeur de la surface de contact entre l'anneau gastrique et l'estomac doit être suffisante pour ne pas concentrer la pression exercée sur l'estomac. De préférence cette largeur est au moins de 1,5 à 2cm.

Pour faciliter la mise en traction du lien permettant le repliement, sous forme d'une boucle , de l'élément longiforme de l'anneau gastrique, il est préférable de mettre en oeuvre un ancillaire 26 particulièrement adapté. Cet ancillaire 26 se présente par exemple sous la forme d'un tube 27, muni d'une poignée 28, laquelle est équipée d'un système mécanique 29 , notamment à molette 30, à roulement ou à cliquet, qui est en prise avec les bouts libres du lien passant à l'intérieur du tube 27 et qui permet la mise en tension desdits bouts libres. Il suffit ainsi au praticien d'actionner ladite poignée 28 jusqu'à ce que cette mise en tension permette le repliement en boucle de l'élément longiforme jusqu'au rapprochement des parties d'extrémités distale et proximale , avant fixation entre elles de celles-ci.

## Revendications

1. Anneau gastrique du type comprenant :
- un élément longiforme déformable en boucle entre une partie d'extrémité distale et une partie d'extrémité proximale, ledit élément longiforme étant composé d'un maillon proximal (2), d'un maillon distal (4) et de maillons intermédiaires (3), articulés selon des axes de pivotement, et
- des moyens de fermeture aptes à replier sur lui-même l'élément longiforme et à fixer les parties d'extrémités distale et proximale, une fois que celles-ci ont été rapprochées lors du repliement de l'anneau, au moins l'élément longiforme étant dans un matériau résorbable,
chaque maillon (2, 3, 4) comportant:
a) une face intérieure (5) destinée à être en contact avec l'estomac lors de la pose de l'anneau, et
b) deux faces latérales internes dont au moins les portions (6, 6') situées à proximité de la face intérieure (5) sont obliques et radialement convergentes,
en sorte que, lors de la fermeture de l'anneau (1), toutes lesdites portions radiales (6,6') des faces latérales internes sont appliquées les unes contre les autres et les faces intérieures (5) des maillons constituent une surface sensiblement continue de constriction.

2. Anneau gastrique selon la revendication 1, **caractérisé en ce qu'**au moins l'élément longiforme est dans un matériau lentement résorbable sur une période de l'ordre de ou inférieure à 2 ans, de préférence comprise entre 16 et 24 mois.

3. Anneau selon l'une des revendications 1 ou 2, **caractérisé en ce qu'**au moins l'élément longiforme est dans un poly-α-hydroxy acide, de préférence un polyacide lactique, plus particulièrement un poly (L-lactide-co-D,L-lactide).

4. Anneau selon la revendication 3, **caractérisé en ce que** chaque maillon intermédiaire (3) comporte, en saillie sur une première face latérale interne (7'), un épaulement (9) formant axe de pivotement entre ledit maillon et un premier maillon adjacent et, sur la seconde face latérale interne (7), un évidement (8) formant logement pour l'épaulement (9) d'un second maillon adjacent, l'épaulement (9) et l'évidement (8) étant dimensionnés pour être solidarisables par emboîtement.

5. Anneau selon l'une des revendications 1 à 4, **caractérisé en ce que** les moyens de fermeture comportent au moins un lien qui est solidaire d'au moins les parties d'extrémités proximale et distale.

6. Anneau selon la revendication 5, **caractérisé en ce que** le lien est résorbable avec une période de résorption supérieure à la période de résorption de l'élément longiforme.

7. Anneau selon l'une quelconque des revendications précédentes, **caractérisé en ce que** chaque maillon comporte deux trous traversants (12), formant dans l'ensemble des maillons (2, 3, 4) deux alignements de trous, pour un double passage d'un seul lien (24), depuis le maillon proximal (2) jusqu'au maillon distal (4) pour le premier alignement puis du maillon distal (4) jusqu'au maillon proximal (2) pour le second alignement.

8. Anneau selon l'une des revendications 1 à 7, **caractérisé en ce que** les moyens de fermeture comportent des moyens de verrouillage formés , au moins en partie , dans les parties d'extrémités proximale et distale de l'élément longiforme , notamment les maillons proximal et distal, à savoir un élément mâle d'emboîtement dans une partie d'extrémité, un élément femelle d'emboîtement dans l'autre partie d'extrémité.

9. Anneau selon la revendication 8, **caractérisé en ce que** l'élément mâle est un plot (18) et l'élément femelle est un évidement (14).

10. Anneau selon l'une des revendications 5 ou 6, **caractérisé en ce que** la partie d'extrémité proximale, éventuellement le maillon proximal (2), comporte une tubulure (16) de passage du lien ou des deux bouts libres (25,25') du lien (24), conformée pour sa fixation sur un ancillaire (26) d'introduction de l'anneau (1), notamment taraudée pour sa fixation par vissage sur ledit ancillaire (26).

11. Ensemble chirurgical comportant un anneau gastrique selon la revendication 10 dont les moyens de fermeture comportent au moins un lien qui est solidaire d'au moins les parties d'extrémité proximale et distale et un ancillaire (26) d'introduction dudit anneau, constitué d'un tube (27) muni d'une poignée (28), laquelle est équipée d'un système mécanique (29), notamment à molette (30), à enroulement ou à cliquet, permettant la mise en tension du lien ou des deux bouts libres du lien.

## Claims

1. A gastric ring (1) of the type comprising :
- an elongate element that is deformable into a loop between a distal end portion and a proximal end portion, said elongate element is being made up of a proximal link (2), a distal link (4), and intermediate links (3) hinged about pivot axes, and
- closure means suitable for folding said elongate element into a loop and for securing the distal and proximal end portions to each other once they have been brought close together by looping the ring, at least the elongate element is being made of a resorbable material,
each link (2, 3, 4) comprising:
a) an inner face (5) for coming into contact with the stomach when the ring is put into place, and
b) two internal end faces in which at least the portions (6, 6') that are situated close to the inner face (5) are oblique and radially convergent,
so that, during closure of the ring (1), all of said radial portions (6, 6') of the internal end faces are pressed against one another and the inner faces (5) of the links constitute a substantially continuous constriction surface.

2. A gastric ring according to claim 1, wherein at least the elongate element is made of a material that is resorbable slowly over a period of about or less than 2 years, and preferably lying in the range 16 months to 24 months.

3. A ring according to any of the claims 1 or 2, wherein at least the elongate element is made of a poly-[alpha]-hydroxy acid, preferably a lactic polyacid, and more particularly a poly (L-lactide-co-D,L-lactide).

4. A ring according to claim 3, wherein each intermediate link (3) includes, projecting from a first internal end face (7'), a shoulder (9) forming a pivot axis between said link and a first adjacent link, and in its second internal end face (7), a recess (8) forming a housing (9) for the shoulder of a second adjacent link, the shoulder (9) and the recess (8) being of dimensions to be suitable for being mutually interengaged.

5. A ring according to any of the claims 1 to 4, wherein the closure means comprise at least one tie secured to at least one of the proximal and distal end portions.

6. A ring according to claim 5, wherein the tie is resorbable with a resorption period that is longer than the resorption period of the elongate element.

7. A ring according to any of the claims 1 to 6, wherein each link has two through holes (12), together forming in the set of links (2, 3, 4) two alignments of holes, for two passes of a single tie (24), from the proximal link (2) to the distal link (4) along the first alignment and then from the distal link (4) to the proximal link (2) along the second alignment.

8. A ring according to any of the claims 1 to 7, wherein the closure means comprise locking means formed, at least in part, in the proximal and distal end portions of the elongate element, notably in the proximal and distal links, namely a male engagement element in one end portion and a female engagement element in the other end portion.

9. A ring according to claim 8, wherein the male element is a stud (18) and the female element is a recess (14).

10. A ring according to claim 5 or 6, wherein the proximal end portion, possibly the proximal link (2), includes a tube (16) fitting for passing the tie or the two free ends (25, 25') of the tie (24), the tube fitting being shaped for securing to an ancillary (26) for introducing the ring (1), and in particular being threaded for screw-fastening to said ancillary (26).

11. Surgical set comprising a ring according to claim 10, said ring having closure means comprising at least one tie secured to at least one of the proximal and distal end portions, and an ancillary (26) for introducing the ring (1), said ancillary (26) is being constituted by a tube (27) provided with a handle (28), which is fitted with a mechanical system (29), in particular a winding or ratchet wheel (30), enabling the tie or the two free ends of the tie to be put under tension.

## Patentansprüche

1. Magenring vom Typ umfassend:
- ein längliches Element, das zwischen einem distalen Endteil und einem proximalen Endteil zu einer Schlaufe verformbar ist, wobei das längliche Element aus einem proximalen Glied (2), einem distalen Glied (4) und aus Zwischengliedern (3) besteht, die um Schwenkachse angesenkt sind, und
- Verschlußmittel, die geeignet sind, das längliche Element auf sich selbst umzubiegen und das distale und das proximale Endteil zu befestigen, sobald diese beim Umbiegen des Rings einander genähert worden sind, wobei wenigstens das längliche Element aus einem resorbierbaren Material besteht,
wobei jedes Glied (2, 3, 4) umfaßt:
a) eine Innenseite (5), die dazu bestimmt ist, bei Einsetzen des Rings mit dem Magen in Kontakt zu sein, und
b) zwei innere Seitenflächen, von denen wenigstens die in der Nähe der Innenseite (5) gelegenen Abschnitte (6, 6') schräg und radial konvergierend sind,
derart, daß bei Verschließen des Rings (1) alles radialen Abschnitte (6, 6') der inneren Seitenflächen aneinander liegen und die Innenseiten (5) der Glieder eine im wesentlichen durchgehende Einschnürfläche bilden.

2. Magenring nach Anspruch 1, **dadurch gekennzeichnet, daß** wenigstens das längliche Element aus einem Material besteht, das über einen Zeitraum in der Größenordnung von oder weniger als 2 Jahren, vorzugsweise zwischen 16 und 24 Monaten langsam resorbierbar ist.

3. Ring nach einem der Ansprüche 1 oder 2, **dadurch gekennzeichnet, daß** wenigstens das längliche Element aus einer Poly-α-hydroxysäure, vorzugsweise einer Polymilchsäure, insbesondere einer Poly (L-lactide-co-D,L-lactide) besteht.

4. Ring nach Anspruch 3, **dadurch gekennzeichnet, daß** jedes Zwischenglied (3) an einer ersten inneren Seitenfläche (7') vorspringend eine Schulter (9), die eine Schwenkachse zwischen dem Glied und einem ersten benachbarten Glied bildet, sowie an der zweiten inneren Seitenfläche (7) eine Ausnehmung (8) umfaßt, die eine Aufnahme für die Schulter (9) eines zweiten benachbarten Glieds bildet, wobei die Schulter (9) und die Ausnehmung (8) bemessen sind, um durch Ineinanderstecken fest miteinander verbindbar zu sein.

5. Ring nach einem der Ansprüche 1 bis 4, **dadurch gekennzeichnet, daß** die Verschlußmittel wenigstens ein Bindeglied umfassen, das wenigstens mit dem proximalen und dem distalen Endteil fest verbunden ist.

6. Ring nach Anspruch 5, **dadurch gekennzeichnet, daß** das Bindeglied resorbierbar ist, mit einem Resorptionszeitraum, der länger als der Resorptionszeitraum des länglichen Elements ist.

7. Ring nach einem der vorhergehenden Ansprüche, **dadurch gekennzeichnet, daß** jedes Glied zwei durchgehende Löcher (12) aufweist, die in allen Gliedern (2, 3, 4) zwei Fluchtlinien von Löchern für einen zweifachen Durchgang eines einzigen Bindegliedes (24), von dem proximalen Glied (2) bis zum distalen Glied (4) für die erste Fluchtlinie, dann vom distalen Glied (4) bis zum proximalen Glied (2) für die zweite Fluchtlinie, bilden.

8. Ring nach einem der Ansprüche 1 bis 7, **dadurch gekennzeichnet, daß** die Verschlußmittel Verriegelungsmittel umfassen, die wenigstens teilweise in dem proximalen und dem distalen Endteil des länglichen Elements, insbesondere dem proximalen und dem distalen Glied, ausgebildet sind, und zwar ein einzusteckendes Steckelement in einem Endteil, ein aufnehmendes Steckelement in dem anderen Endteil.

9. Ring nach Anspruch 8, **dadurch gekennzeichnet, daß** das einzusteckende Element ein Stift (18) und das aufnehmende Element eine Ausnehmung (14) ist.

10. Ring nach einem der Ansprüche 5 oder 6, **dadurch gekennzeichnet, daß** das proximale Endteil, eventuell das proximale Glied (2), einen Stutzen (16) für den Durchgang des Bindegliedes oder der beiden freien Enden (25, 25') des Bindegliedes (24) umfaßt, der für seine Befestigung an einem Zubehör (26) zum Einführen des Rings (1) ausgebildet, insbesondere mit einem Innengewinde für seine Schraubbefestigung an dem Zubehör (26) versehen ist.

11. Chirurgische Anordnung, die einen Magenring nach Anspruch 10 umfaßt, dessen Verschlußmittel wenigstens ein Bindeglied, das wenigstens mit dem proximalen und dem distalen Endteil fest verbunden ist, und ein Zubehör (26) zum Einführen des Rings umfassen, das von einem Rohr (27) gebildet ist, welches mit einem Griff (28) versehen ist, der mit einem mechanischen Wickel- oder Sperrklinkensystem (29), insbesondere mit Rädchen (30), ausgestattet ist, das das Spannen des Bindegliedes oder der beiden freien Enden des Bindegliedes ermöglicht.
